# EUROPEAN PATENT APPLICATION

(11) **EP 1 600 170 A1**
(43) Date of publication of application: **30.11.2005**
(21) Application number: 04714460.5
(22) Date of filing: 25.02.2004
(51) Int. Cl.: A61K 47/36, A61K 9/72, A61K 9/12, A61K 9/14, A61K 45/00, A61K 31/731, A61P 11/00, A61P 11/06, A61P 43/00

(54) **SUSTAINED-RELEASE PHARMACEUTICAL COMPOSITION FOR LUNG ADMINISTRATION**

(30) Priority: 26.02.2003 JP 2003049019
(71) Applicant: OTSUKA PHARMACEUTICAL CO., LTD., Chiyoda-ku Tokyo 101-8535 (JP)
(72) Inventor: Yamamoto, Akira, Forumu Horikawa Imadegawa 205, Kamigyo-ku, Kyoto-shi, Kyoto 6020054 (JP); YAMADA, Keigo, Tokushima 7700872 (JP)
(74) Representative: HOFFMANN EITLE
(86) International application number: PCT/JP2004/002193
(87) International publication number: WO 2004/075920

(57) **Abstract**

The present invention provides a sustained-release pharmaceutical composition containing a pharmacologically active substance, the pharmaceutical composition being able to control the pulmonary absorption of the pharmacologically active substance and produce prolonged pharmacological effects when administered via the pulmonary route. The invention provides a pharmaceutical composition containing carrageenan and a pharmacologically active substance that can be administered via the lungs, a production method and use thereof.

## Description

### TECHNICAL FIELD

The present invention relates to a sustained-release pharmaceutical composition that can be administered pulmonarily, and a production method and use thereof.

### BACKGROUND OF THE INVENTION

Generally, pulmonary absorption of drugs is faster than gastrointestinal absorption and it is known that the smaller the molecular weight and the higher the oil/water partition coefficient, the faster the absorption. In recent years, it has been reported that comparatively fast absorption rates can be achieved even with water-soluble drugs and macromolecular drugs. Therefore, pulmonary absorption is attracting attention as a new administration route for drugs expected to exhibit systemic effects such as insulin, calcitonin and like biologically active peptides.

However, some drugs, even administered in trace amounts, have a strong action. If pulmonary absorption is too rapid, the drug concentration in plasma rapidly increases and may produce adverse side effects. Furthermore, although pulmonary administration has the advantages of allowing good absorption and immediate effectiveness, it is difficult to prolong the therapeutic effects of drugs and there is also the problem of patient inconvenience because some drugs require frequent administration.

A means for prolonging the pharmacological effects of inhalants is, for example, the use of a liposome preparation as a liquid inhalant as described in U.S. Patent No. 5192528. However, liposomes are generally unstable and difficult to preserve stably at room temperature for a long period of time.

Therefore, a stable sustained-release pharmaceutical composition for pulmonary administration that allows pharmacologically active substances to be absorbed in the lungs at a controlled rate and exhibits prolonged pharmacological effects has been in great demand.

Japanese Unexamined Patent Publication No. 36233/1992 discloses a sustained-release drug prepared by producing microspheres from an aqueous solution of a water-soluble polymer containing a physiologically active substance and encapsulating the polymer in a hydrophobic, biodegradable and bioabsorbable polymer. As examples of such water-soluble macromolecular substance, the publication mentions sodium alginate, gelatin, carrageenan, etc. However, there is no mention about the administration of this sustained-release drug via the pulmonary route.

### DISCLOSURE OF THE INVENTION

An object of the invention is to provide a sustained-release pharmaceutical composition containing a pharmacologically active substance, the pharmaceutical composition being able to control the pulmonary absorption of the pharmacologically active substance and produce prolonged pharmacological effects when administered via the pulmonary route.

The present inventors carried out intensive research to solve the problem in the background art and found that addition of carrageenan to a pharmacologically active substance that can be administered via the lungs can solve the problem. The inventors carried out further intensive research based on this finding and thereby accomplished the invention.

The present invention thus provides the following:
Item 1. A sustained-release pharmaceutical composition for pulmonary administration comprising carrageenan and a pharmacologically active substance that can be administered via the lungs.
Item 2. A pharmaceutical composition according to item 1, wherein the carrageenan is at least one member selected from the group consisting of kappa-carrageenan, iota-carrageenan and lambda-carrageenan.
Item 3. A pharmaceutical composition according to item 1, wherein the pharmacologically active substance is at least one member selected from the group consisting of anticholinergic drugs, β2 stimulants, steroids, antiasthmatic drugs, antiallergic drugs, antiinflammatory drugs, antibacterial drugs, antifungal drugs, anti-influenza virus drugs, peptide drugs, antitumor drugs and vitamins.
Item 4. A pharmaceutical composition according to item 1, wherein carrageenan is present in an amount of about 0.001 to 10⁷ wt.% relative to the pharmacologically active substance.
Item 5. A pharmaceutical composition according to item 1, wherein carrageenan mainly comprising iota-carrageenan is present in an amount of about 0.01 to 10⁵ wt.% relative to the pharmacologically active substance and the pharmacologically active substance is one member selected from the group consisting of β2 stimulants, antiasthmatic drugs and steroids.
Item 6. A pharmaceutical composition according to item 1, which is in the form of a powder having a mean particle diameter of about 0.1 to 20 µm.
Item 7. A process for preparing a sustained release pharmaceutical composition in the form of a powder for pulmonary administration, the process comprising drying an aqueous solution or aqueous dispersion containing a pharmacologically active substance that can be administered via the lungs and carrageenan, followed by pulverization, or comprising spray drying the solution or dispersion.
Item 8. An inhalant comprising the pharmaceutical composition according to any one of items 1 to 6.
Item 9. An aerosol comprising the pharmaceutical composition according to any one of items 1 to 6, a propellant and an aerosol container.
Item 10. An aerosol according to item 9, wherein relative to the pharmacologically active substance, carrageenan is present in an amount of about 0.01 to 10⁴ wt.%, and propellant is present in an amount of about 10² to 10⁷ wt.%.
Item 11. An aerosol according to item 9, which further comprises at least one member selected from the group consisting of solvents and dispersants.
Item 12. An aerosol according to item 11, wherein relative to the pharmacologically active substance, the solvent is present in an amount of about 0 to 10⁶ wt.%, and the dispersant is present in an amount of about 0 to 10³.
Item 13. A method for administering to a patient a pharmacologically active substance that can be administered via the lungs, the method comprising administering an effective amount of a pharmacologically active substance in combination with carrageenan to the patient via the pulmonary route.
Item 14. A method for sustainedly releasing a pharmacologically active substance in the lungs, comprising administering via the lungs an effective amount of a pharmaceutical composition containing the pharmacologically active substance and carrageenan.
Item 15. Use of carrageenan for preparing a sustained-release pharmaceutical composition for pulmonary administration of a pharmacologically active substance.
Item 16. Use of carrageenan for sustainedly releasing a pharmacologically active substance in the lungs by administering via the lungs an effective amount of a pharmaceutical composition containing the pharmacologically active substance.

### BRIEF DESCRIPTION OF THE DRAWINGS

Fig. 1 is a graph showing the change in serum theophylline concentration after intratracheal and intravenous administrations of solution A used in Test Example 1 (n=4, mean ± S.D.).
Fig. 2 is a graph showing the change in serum theophylline concentration after intratracheal administration of solutions A to D used in Test Example 1 (n=4, mean ± S.D.).

### DETAILED DESCRIPTION OF THE INVENTION

The sustained-release pharmaceutical composition for pulmonary administration (hereinafter also referred to simply as "pharmaceutical composition") of the invention is described below in detail.

The pharmaceutical composition of the invention comprises a pharmacologically active substance that can be administered via the lungs (hereinafter also referred to simply as "pharmacologically active substance") and carrageenan. The composition is characterized by containing carrageenan as a means for controlling the absorption rate of the pharmacologically active substance in the lungs.

The pharmacologically active substance used in the invention is not particularly limited so long as the substance exhibits pharmacological effects when administered to, for example, mammals via the pulmonary route. A wide variety of known substances that act locally, for example, on the trachea, bronchi, lungs or the like, or act on the entire body can be used. Examples of such pharmacologically active substances include central nervous system drugs, peripheral nervous system drugs, cardiovascular drugs, drugs for the digestive organs, antibiotics, chemotherapeutic drugs and the like. Specific examples include anticholinergic drugs (e.g., ipratropium bromide, flutropium bromide, oxitropium bromide, thiotropium bromide), β2 stimulants (e.g., procaterol, fenoterol, salbutamol, formoterol, salmeterol), steroids (e.g., beclomethasone, fluticasone, budesonide), antiasthmatic drugs (e.g., theophylline, aminophylline, ozagrel), antiallergic drugs (e.g., ketotifen, terfenadine, azelastine, epinastine), antiinflammatory drugs (e.g., diclofenac sodium, ibuprofen, indomethacin), antibacterial drugs (e.g., cefixime, cefdinir, ofloxacin, tosufloxacin), antifungal drugs (e.g., fluconazole, itraconazole), anti-influenza virus drugs (e.g., zanamivir, oseltamivir, amantadine), peptide drugs (e.g., insulin, calcitonin), antitumor drugs (e.g., fluorouracil, gefitinib), vitamin tablets (e.g., alfacalcidol, mecobalamin) and the like; and derivatives thereof. Such pharmacologically active substances can be used alone or as a mixture of at least two members selected from the above group. Such pharmacologically active substances include salts (e.g., hydrochlorides) and prodrugs thereof.

Carrageenan used in the invention is a macromolecule having a molecular weight of 100,000 to 500,000 and obtained by extracting with water red algae such as Gigartinaceae *Gigartina* and *Chondrus* and Solieriaceae *Eucheuma.* Carrageenan is a polysaccharide mainly comprising galactose and 3,6-anhydrogalactose. As shown below, carrageenan has sulfate hemiester moieties in the unit structure and may be classified into five types, i.e., kappa(κ)-carrageenan, iota(τ)-carrageenan, lambda(λ)-carrageenan, mu(µ)-carrageenan and eta(η)-carrageenan, according to the content of sulfate hemiester moieties. In the invention, the five types of carrageenan can be used singly or in combination. The mixing ratio can be suitably selected according to the purpose of use. Of the above five types of carrageenan, kappa-carrageenan, iota-carrageenan and lambda-carrageenan are preferable. Especially preferable is carrageenan mainly comprising iota-carrageenan. iota-carrageenan is particularly suitable for use.

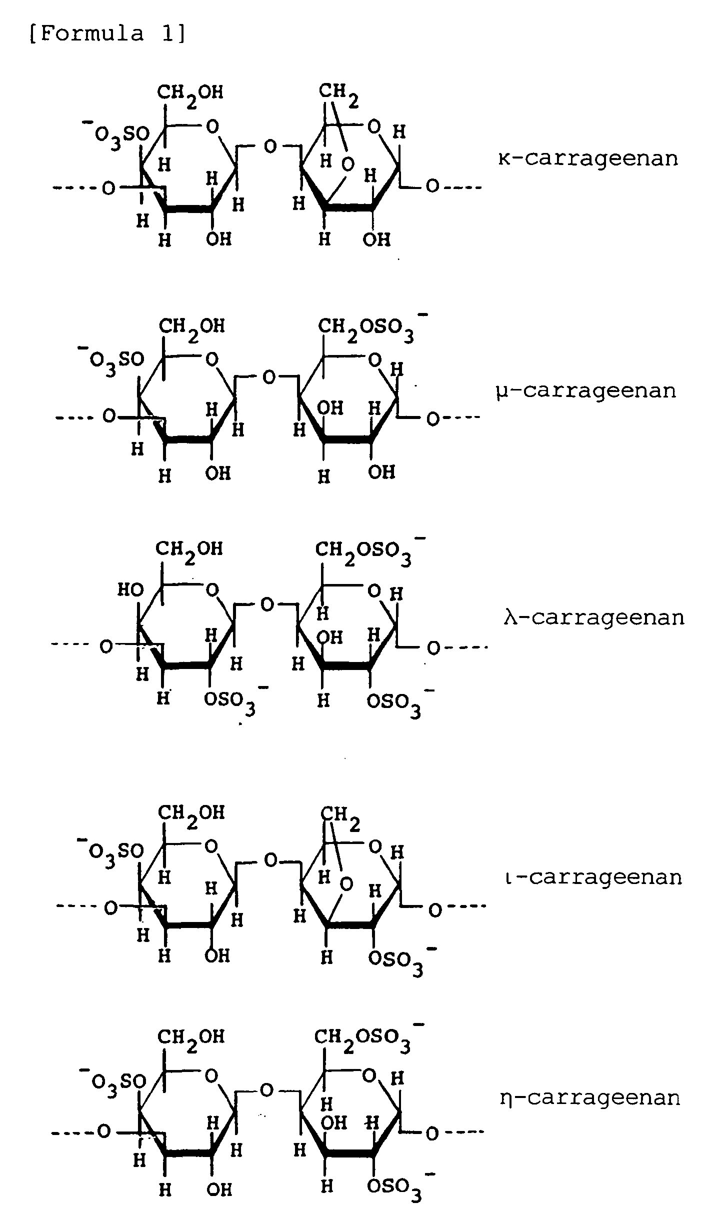

The pharmaceutical composition of the invention contains the above pharmacologically active substance and carrageenan. When the composition is administered via the pulmonary route, the pharmacologically active substance is retained in the lungs by the action of carrageenan and gradually and sustainedly released. Therefore, the pulmonary absorption rate of the pharmacologically active substance can be controlled and the plasma pharmacologically active substance concentration is maintained sustainedly and stably. Moreover, the pharmaceutical composition of the invention has good storage stability.

The pharmaceutical composition of the invention may contain, in addition to the pharmacologically active substance and carrageenan, additives as described below. Such additives are not particularly limited and may be any additives used in the preparation of drugs. Specific examples thereof include solid excipients such as refined sugar, lactose, glucose, fructose, sucrose, mannitol, sorbitol, arabinose, xylitol, dextrose and the like; liquid excipients such as propylene glycol and like inert liquids; binders such as methylcellulose, hydroxypropylcellulose, polyvinylpyrrolidone, polyethylene glycols, refined sugar and the like; lubricants such as magnesium stearate, light anhydrous silicic acid, talc, sodium lauryl sulfate and the like; preservatives such as sodium benzoate, sodium hydrogen sulfite, methyl paraben, propyl paraben and the like; stabilizers such as citric acid, sodium citrate and the like; dispersants such as methylcellulose, polyvinylpyrrolidone, lecithin, sorbitan trioleate, oleyl alcohol, polyoxyethylene sorbitan fatty acid esters and like suspension agents or surfactants; solvents such as water, ethanol, 1-propanol, 2-propanol and the like; isotonic agents such as sodium chloride and the like; pH adjusters such as sulfuric acid, hydrochloric acid and the like; etc. The kinds and amounts of additives can be suitably selected according to the purpose of use.

The components and their proportions in the pharmaceutical composition of the invention may be suitably selected according to the targeted disease, age and gender of the patient, condition of the disease, etc.

For example, the pharmaceutical composition of the invention may contain carrageenan in an amount of about 0.001 to 10⁷ wt.% (preferably about 0.01 to 10⁶ wt.%, more preferably about 0.1 to 10⁵ wt.%, particularly preferably about 1 to 10⁴ wt.%), relative to the weight of the pharmacologically active substance. Additives may be incorporated in the composition in an amount of about 0 to 10⁷ wt.% (preferably about 0.5 to 10⁶ wt.%, more preferably about 1 to 10⁴ wt.%, particularly preferably about 10 to 10³ wt.s), relative to the weight of the pharmacologically active substance.

According to one preferred embodiment of the invention, the pharmaceutical composition contains carrageenan (particularly carrageenan mainly comprising iota-carrageenan) in an amount of about 0.01 to 10⁵ wt.% (preferably about 0.1 to 10⁴ wt.%), and additives (particularly water, ethanol, etc.) in an amount of about 0 to 10⁷ wt.% (preferably about 10 to 10⁶ wt.%), relative to the weight of pharmacologically active substance (particularly β2 stimulants, antiasthmatic drugs and steroids).

According to a further preferred embodiment of the invention, the pharmaceutical composition contains iota-carrageenan in an amount of about 0.1 to 10⁴ wt.% (preferably about 1 to 10³ wt.%), and additives (particularly water, ethanol, etc.) in an amount of about 1 to 10⁶ wt.% (preferably about 10 to 10⁵ wt.%), relative to the weight of pharmacologically active substance (particularly theophylline, procaterol, etc.).

According to another preferred embodiment of the invention, the pharmaceutical composition contains carrageenan (particularly carrageenan mainly comprising iota-carrageenan) in an amount of about 0.1 to 10⁴ wt.% (preferably about 1 to 10³ wt.%), relative to the weight of pharmacologically active substance (particularly β2 stimulants, antiasthmatic drugs and steroids).

According to a further preferred embodiment of the invention, the pharmaceutical composition contains iota-carrageenan in an amount of about 0.1 to 10³ wt.% (preferably about 1 to 10² wt.%), relative to the weight of pharmacologically active substance (particularly theophylline, procaterol, etc.).

The pharmaceutical composition of the invention for pulmonary administration is usually used as an inhalant. The composition can be formed into dry powder inhalants, inhalant suspensions, inhalant solutions, encapsulated inhalants and like known forms of inhalants. Such forms of inhalants can be prepared by filling the pharmaceutical composition of the invention into an appropriate inhaler such as a metered-dose inhaler, dry powder inhaler, atomizer bottle, nebulizer etc. before use. Of the above forms of inhalants, powder inhalants are particularly preferable.

When the pharmaceutical composition of the invention is used in the form of a powder, the mean particle diameter of the powder is not especially limited but, in view of the residence of the particles in the lungs, is preferably in the range of about 0.1 to 20 µm, and particularly about 1 to 5 µm. Although the particle size distribution of the powder pharmaceutical composition of the invention is not particularly limited, it is preferable that particles having a size of about 25 µm or more account for not more than about 5% of the particles, and particularly preferably 1% or less.

The pharmaceutical composition in the form of a powder of the invention can be produced by, for example, the drying-micronization method, the spray drying method and the like.

According to the drying-pulverization method, the pharmaceutical composition in the form of a powder can be prepared by drying an aqueous solution (or aqueous dispersion) containing a pharmacologically active substance and carrageenan and microparticulating the dried product. Stated more specifically, after dissolving (or dispersing) carrageenan in an aqueous medium, a pharmacologically active substance is added and dissolved (or dispersed) by stirring using a homogenizer, etc. to give an aqueous solution (or aqueous dispersion). The aqueous medium may be water alone or a mixture of water and a lower alcohol. Examples of usable lower alcohols include methanol, ethanol, 1-propanol, 2-propanol and like water-miscible alcohols. Ethanol is particularly preferable. After the obtained aqueous solution (or aqueous dispersion) is dried by blower, lyophilization, etc., the resulting product is pulverized or microparticulated into fine particles using jet mills, ball mills or like devices to give a powder having the above mean particle diameter. If necessary, additives as mentioned above may be added in any of the above steps.

According to the spray-drying method, the pharmaceutical composition in the form of a powder of the invention can be prepared, for example, by spray-drying an aqueous solution (or aqueous dispersion) containing a pharmacologically active substance and carrageenan for microparticulation. The aqueous solution (or aqueous dispersion) can be prepared following the procedure of the above drying-micronization method. The spray-drying process can be performed using a known method, thereby giving a powdery pharmaceutical composition in the form of globular particles with the above-mentioned mean particle diameter.

The inhalant suspensions, inhalant solutions, encapsulated inhalants, etc. can also be prepared using the pharmaceutical composition in the form of a powder produced by the drying-micronization method, the spray-drying method and the like, or by using carrageenan and a pharmacologically active substance that can be administered via the lungs, according to known preparation methods.

Furthermore, the inhalant comprising the pharmaceutical composition of the invention is preferably used as an aerosol. The aerosol can be prepared, for example, by filling the pharmaceutical composition of the invention and a propellant into an aerosol container. If necessary, dispersants, solvents and the like may be added. The aerosols may be prepared as 2-phase systems, 3-phase systems and diaphragm systems (double containers). The aerosol can be used in any form of a powder, suspension, solution or the like.

Examples of usable propellants include liquefied gas propellants, compressed gases and the like. Usable liquefied gas propellants include, for example, fluorinated hydrocarbons (e.g., CFC substitutes such as HCFC-22, HCFC-123, HFC-134a, HFC-227 and the like), liquefied petroleum, dimethyl ether and the like. Usable compressed gases include, for example, soluble gases (e.g., carbon dioxide, nitrous oxide), insoluble gases (e.g., nitrogen) and the like.

The dispersant and solvent may be suitably selected from the additives mentioned above.

The aerosol can be prepared, for example, by a known 2-step method comprising the step of preparing the composition of the invention and the step of filling and sealing the composition and propellant into the aerosol container.

As a preferred embodiment of the aerosol according to the invention, the following aerosol can be mentioned: Examples of usable pharmacologically active substances include procaterol, theophylline, steroids and salts thereof (hydrochlorides, sulfates, etc.). Theophylline and procaterol (or hydrochlorides thereof) are particularly preferable. As carageenan, those mainly comprising iota-carrageenan are preferable. As propellants, fluorinated hydrocarbons such as HFC-134a, HFC-227 and like CFC substitutes are preferable. Examples of usable solvents include water, ethanol, 2-propanol and the like. Water and ethanol are particularly preferable. In particular, a weight ratio of water to ethanol in the range of about 0:1 to 10:1 may be used.

The aerosol of the invention contains carrageenan in an amount of about 0.01 to 10⁴ wt.% (preferably about 0.1 to 10³ wt.%), propellant in an amount of about 10² to 10⁷ wt.% (preferably about 10³ to 10⁶ wt.%), solvent in an amount of about 0 to 10⁶ wt.% (preferably about 10 to 10⁵ wt.%), and dispersant in an amount of 0 to 10³ wt.% (preferably about 0.01 to 10² wt.%), relative to the weight of pharmacologically active substance.

The pharmaceutical compositions of the invention are safe and effective for lung diseases and systemic diseases in mammals (e.g., humans, mice, rats, cats, dogs, sheep, horses, cows, monkeys) and can maintain therapeutic effectiveness for a long time. The compositions can be used, for example, as anti-asthmatic agents, antiallergic agents, eosinophil chemotaxis inhibitors and preventive and therapeutic agents for diseases associated with eosinophilic infiltration (e.g., urticaria, atopic dermatitis, allergic rhinitis, hypersensitivity pneumonitis and like allergic diseases, eczema, dermatitis herpetiformis, psoriasis and like skin diseases, eosinophilic pneumonia (PIE syndrome), chronic obstructive pulmonary disease (COPD) and like respiratory diseases) and infectious diseases caused by various bacteria. The compositions are particularly useful as anti-asthmatic agents and preventive and therapeutic agents for chronic obstructive pulmonary disease (COPD), hypersensitivity pneumonitis, eosinophilic pneumonia and influenza or like infectious diseases.

Although the dosage of the pharmaceutical composition of the invention may vary depending on the type of pharmacologically active substance, the targeted disease, age, body weight, condition of the disease, administration route, number of administration times, etc., the composition is administered in an amount effective for allowing the pharmacologically active substance to exhibit its pharmacological effects. For example, the composition is preferably administered such that the active ingredient (pharmacologically active substance) can be given to a human adult in a dose of about 0.001 to about 100 mg, and given in a single dose or twice-four times a day.

The form of the pharmaceutical composition of the invention such as a powder, solution, suspension etc. may be suitably selected according to the type of pharmacologically active substance, the targeted disease etc.

As an administration route, direct inhalation via the mouth using an inhaler is usually preferable.

Since the pharmaceutical composition of the invention allows direct local administration into the airways, the pharmacologically active substances contained therein produce immediate effects. Furthermore, as it contains carrageenan, the composition enables the sustained release of the pharmacologically active substance in the lungs, and exhibition of prolonged pharmacological effects. Therefore, when the pharmaceutical composition of the invention is administered via the lungs, frequent administration is unnecessary, and patient inconvenience is reduced. In addition, the administration of the minimum dose required can largely reduce the risk of adverse side effects caused by massive drug doses. Thus the pharmaceutical composition of the invention can enhance therapeutic effectiveness of the drug and reduce adverse side effects.

### BEST MODE FOR CARRYING OUT THE INVENTION

Examples are given below to illustrate the invention in more detail, but the scope of the invention is not limited to these examples.

### Example 1

(1) 15 mg of procaterol hydrochloride (mean particle diameter 1.8 µm), (2) 100 mg of iota-carrageenan (product No. C-1138, produced by Sigma Chemical Co. and sold by Sigma Aldrich Japan K.K.), (3) 87.90 g of HFC-134a (product No. D10252125, produced and sold by Du Pont-Mitsui Fluorochemicals Co., Ltd.), (4) 2.0 g of ethyl alcohol (99.5%), and (5) 10.0 g of water were mixed in the following manner to give an inhalant suspension.

First, the carrageenan was dissolved in water heated to about 80°C and the solution was cooled to about 40°C. After addition of the procaterol hydrochloride, the mixture was stirred using a homogenizer. While cooling the mixture at about -30°C, 20 g of HFC-134a was added with stirring and then the ethyl alcohol was added with stirring. HFC-134a was further added with stirring to make the total weight of the mixture about 100 g. A 9 g portion of the resulting mixture was filled into an aerosol container and the container was furnished with a metering valve for releasing the aerosol in an amount of 50 µl each time.

### Example 2

1.0 g of iota-carrageenan (product No. C-1138, produced by Sigma Chemical Co. and sold by Sigma Aldrich Japan K.K.) was dissolved in 100 g of water heated to about 80°C and the solution was cooled to about 40°C. 20 g of theophylline was added and dissolved by stirring using a homogenizer. The resulting solution was dried with a blower drying machine (SPHH-200, manufactured by Tabai Espec Corp.) and pulverized to about 2 µm with a spiral jet mill ("50AS", manufacture by Hosokawa Micron Corp.). A 10-fold weight of lactose was added thereto and mixed using a drum mixer to give an inhalant powder.

### Test Example 1

The following four types of pharmaceutical solutions were prepared using theophylline as a pharmaceutically active compound. Solution B is a composition of the invention, whereas solutions A, C and D are comparative compositions.

Solution A: Theophylline was dissolved in isotonic phosphate buffer (PBS(-), Product No. 05913, manufactured by Nissui Pharmaceutical Co., Ltd.) to give a 1 mg/ml theophylline solution A.

Solution B: Iota-carrageenan (product No. C-1138, produced by Sigma Chemical Co. and sold by Sigma Aldrich Japan K.K.) was dissolved in solution A heated to about 80°C, and the resulting solution was cooled to room temperature to give a 1 w/v% (=10 mg/ml) iota-carrageenan solution B.

Solution C: Gelatin (product No. 16631-92, produced and sold by Nacalai Tesque, Inc.) was dissolved in solution A heated to about 80°C, and the resulting solution was cooled to room temperature to give a 5 w/v% gelatin solution C.

Solution D: Sodium alginate with a viscosity of 500-600 cP (product No.199-09961, manufactured by Wako Pure Chemical Industries, Ltd.) was dissolved in solution A to give a 2 w/v% sodium alginate solution D.

Wistar male rats (body weight about 220 g) fasted for about 18 hours were used as experimental animals. The intratracheal administration of the solutions was performed according to the method described in Schanker, L. S. et al., Am. J. Physiol., 222 (1972), p.409. That is, the rats were anesthetized with pentobarbital (about 32 mg/kg) and secured on their back on animal boards, the tracheas were exposed through a longitudinal incision. A section of polyethylene tube (i.d. 1.5 mm, o.d. 2.3 mm) 2.5 cm in length was inserted through the tracheal incision for a distance of 0.6 cm between the forth and fifth tracheal rings caudal to the thyroid cartilage. Each of one hundred microliters of drug solutions A-D was drawn into a glass microsyringe, and then rat was maintained at an angle of 80°. The tip of syringe was inserted through the polyethylene tube to 1-2 mm above the bifurcation of the trachea. Then, the solution was injected over a period of 1-2 sec. About 45 sec after the administration, the rat was positioned at an angle of 10°and the incision in the skin was then closed with suture.

After this operation, intravenous administration was performed by administering 100 µl of solution A through the femoral vein.

0.2 ml of blood was collected from the subclavian vein at 15 sec., 30 sec., 1 min., 2 min., 5 min., 10 min., 15 min, 30 min., 1 hr., 1.5 hr., 2 hr., 2.5 hr., 3 hr., 4 hr., 5 hr. and 6 hr. after the intratracheal and intravenous administrations and centrifuged at 1800 g for 10 minutes. The theophylline concentrations in the obtained serum were measured by HPLC.

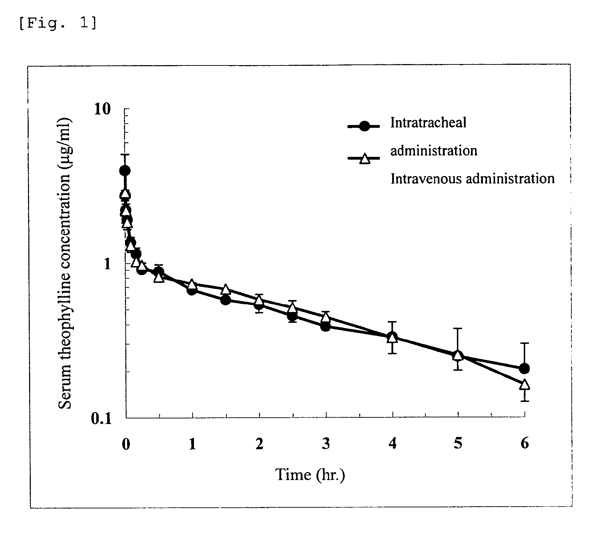

Fig. 1 shows the serum concentration-time profiles of theophylline after intratracheal and intravenous administrations of solution A (n=4, mean ± S.D.).

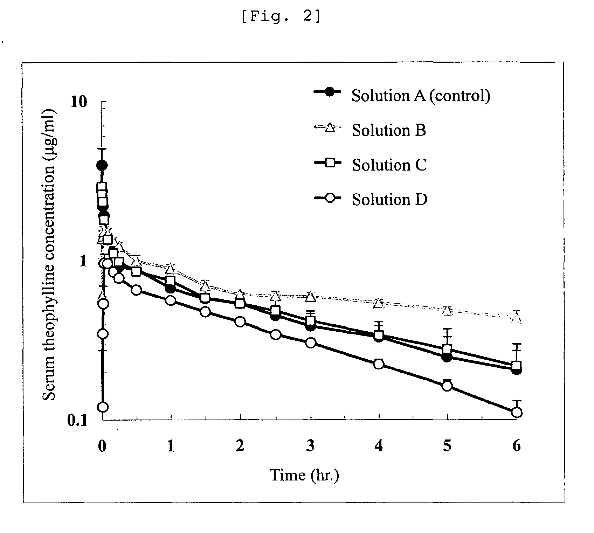

Fig. 2 shows the serum concentration-time profiles of theophylline after intratracheal administration of solutions A to D (n=4, mean ± S.D.).

Table 1 shows the mean pharmacokinetic parameters for the intratracheal administration of solutions A to D at a dose of 100 µg/body (n=4). The parameters in Table 1 indicate the following:
- AUC₆ₕᵣ:: Area under the serum theophylline concentration-time curve to 6 hrs after administration (µg·hr/ml)
- AUC_{inf}:: Area under the serum theophylline concentration-time curve to infinite time after administration (µg·hr/ml)
- Cₘₐₓ:: Maximum serum theophylline concentration (µg/ml)
- Tₘₐₓ:: Time required to reach maximum serum theophylline concentration (hr)
- MRT_{inf}:: Mean residence time (calculated through infinite time after administration) (hr)
- MAT_{inf}:: Mean absorption time (calculated through infinite time after administration) (hr)

Fig. 1 shows that pulmonary absorption of theophylline was as quick and effective as achieved by intravenous administration.

As is clear from Fig. 2 and Table 1, as compared with carrageenan-free solution A (control), the serum theophylline concentration change after the intratracheal administration of 1 w/v% iota-carrageenan solution B shows sustained release pharmacokinetic properties, i.e., a 5.5-fold increase in time to reach the maximum serum theophylline concentration (Tₘₐₓ), 2.2-fold increase in mean residence time (MRT_{inf}), 4.9-fold increase in mean absorption time (MAT_{inf}) and 40.2% suppression of the maximum concentration without reducing the area under the serum concentration-time curves (AUC₆ₕᵣ, AUC_{inf}). These results indicate that addition of 1 w/v% iota-carrageenan imparts valuable sustained release properties, i.e., prolonged therapeutic effects as demonstrated by maintenance of AUC₆ₕ and AUC_{inf}, reduced adverse side effects as demonstrated by suppression of Cₘₐₓ, and increased effective serum theophylline concentration residence time as demonstrated by increase of MRT_{inf} and MAT_{inf}.

As compared with solution A, 5 w/v% gelatin solution C, whose sustained release effect had been confirmed for 5(6)-carboxyfluorescein (CF)(molecular weight: 376), slightly suppressed Cₘₐₓ but no increase was seen in Tₘₐₓ, MRT_{inf} and MAT_{inf}, and thus no sustained release effects were observed.

Furthermore, 2 w/v% sodium alginate solution D, which has a viscosity similar to 1 w/v% iota-carrageenan solution B, decreased AUC₆ₕ and AUC_{inf} and also reduced MRT_{inf} and MAT_{inf}. It is thus concluded that the pulmonary sustained release effect of carrageenan is not based on its viscosity but is carrageenan-specific.

**[Table 1]**

| Solution | Dose (µg/body) | AUC₆ₕᵣ (µg·hr/mL) | AUC_{inf} (*µ*g·hr/mL) | Cₘₐₓ (*µ*g/mL) | Tₘₐₓ (hr) | MRT_{inf} (hr) | MAT_{inf} (hr) |
|---|---|---|---|---|---|---|---|
| A (Control) | 100 | 2.89 | 4.02 | 3.96 | 0.0042 | 4.43 | 1.32 |
| B (carrageenan) | 100 | 3.96 | 8.33 | 1.59 | 0.023 | 9.55 | 6.44 |
| C (gelatin) | 100 | 3.00 | 4.01 | 2.92 | 0.0042 | 4.29 | 1.18 |
| D (sodium alginate) | 100 | 2.11 | 2.44 | 0.96 | 0.033 | 3.00 | -0.11 |

By using carrageenan together with a pharmacologically active substance, the pharmaceutical composition of the invention can advantageously control the pulmonary absorption rate of the pharmacologically active substance and allow the substance to exhibit prolonged pharmacological effects.

The publications mentioned in this specification are incorporated herein by reference.

## Claims

1. A sustained-release pharmaceutical composition for pulmonary administration comprising carrageenan and a pharmacologically active substance that can be administered via the lungs.

2. A pharmaceutical composition according to claim 1, wherein the carrageenan is at least one member selected from the group consisting of kappa-carrageenan, iota-carrageenan and lambda-carrageenan.

3. A pharmaceutical composition according to claim 1, wherein the pharmacologically active substance is at least one member selected from the group consisting of anticholinergic drugs, β2 stimulants, steroids, antiasthmatic drugs, antiallergic drugs, antiinflammatory drugs, antibacterial drugs, antifungal drugs, anti-influenza virus drugs, peptide drugs, antitumor drugs and vitamins.

4. A pharmaceutical composition according to claim 1, wherein carrageenan is present in an amount of about 0.001 to 10⁷ wt.% relative to the pharmacologically active substance.

5. A pharmaceutical composition according to claim 1, wherein the pharmacologically active substance is one member selected from the group consisting of β2 stimulants, antiasthmatic drugs and steroids, and carrageenan mainly comprising iota-carrageenan is present in an amount of about 0.01 to 10⁵ wt.% relative to the pharmacologically active substance.

6. A pharmaceutical composition according to claim 1, which is in the form of a powder having a mean particle diameter of about 0.1 to 20 µm.

7. A process for preparing a sustained release pharmaceutical composition in the form of a powder for pulmonary administration, the process comprising drying an aqueous solution or aqueous dispersion containing a pharmacologically active substance that can be administered via the lungs and carrageenan, followed by pulverization, or comprising spray drying the solution or dispersion.

8. An inhalant comprising the pharmaceutical composition according to any one of claims 1 to 6.

9. An aerosol comprising the pharmaceutical composition according to any one of claims 1 to 6, a propellant and an aerosol container.

10. An aerosol according to claim 9, wherein relative to the pharmacologically active substance, carrageenan is present in an amount of about 0.01 to 10⁴ wt.%, and propellant is present in an amount of about 10² to 10⁷ wt.%.

11. An aerosol according to claim 9, which further comprises at least one member selected from the group consisting of solvents and dispersants.

12. An aerosol according to claim 11, wherein relative to the pharmacologically active substance, the solvent is present in an amount of about 0 to 10⁶ wt.%, and the dispersant is present in an amount of about 0 to 10³.

13. A method for administering to a patient a pharmacologically active substance that can be administered via the lungs, the method comprising administering an effective amount of a pharmacologically active substance in combination with carrageenan to the patient via the pulmonary route.

14. A method for sustainedly releasing a pharmacologically active substance in the lungs, comprising administering via the lungs an effective amount of a pharmaceutical composition containing the pharmacologically active substance and carrageenan.

15. Use of carrageenan for preparing a sustained-release pharmaceutical composition for pulmonary administration of a pharmacologically active substance.

16. Use of carrageenan for sustainedly releasing a pharmacologically active substance in the lungs by administering via the lungs an effective amount of a pharmaceutical composition containing the pharmacologically active substance.
